# NEUE EUROPÄISCHE PATENTSCHRIFT

(11) **EP 1 051 189 B2**
(45) Veröffentlichungstag und Bekanntmachung des Hinweises auf die Entscheidung über den Einspruch: **14.01.2009**
(45) Hinweis auf die Patenterteilung: 10.08.2005
(21) Anmeldenummer: 99910158.7
(22) Anmeldetag: 27.01.1999
(51) Int. Cl.: A61K 39/00, A61K 9/08, A61K 9/107, A61K 39/39

(54) **Vakzinformulierung enthaltend ein Polykation als Adjuvans sowie Zuckeralkohol und niedrige Konzentrationen anorganischer Ionen**
Vaccine formulation containing a polycation as an adjuvant and sugar-alcohols and low concentrations of anorganic ions
Formulation vaccinale comprenant un polycation en tant qu'adjuvant et des alcohols de sucres et de faibles concentrations d'ions anorganiques

(30) Priorität: 30.01.1998 DE 19803453
(43) Veröffentlichungstag der Anmeldung: 15.11.2000
(73) Patentinhaber: Intercell AG, 1030 Vienna (AT)
(72) Erfinder: BUSCHLE, Michael, A-2345 Brunn/Gebirge (AT); BIRNSTIEL, Max, A-1080 Wien (AT); SCHMIDT, Walter, A-1030 Wien (AT)
(74) Vertreter: Wachenfeld, Joachim
(86) Internationale Anmeldenummer: PCT/EP1999/000524
(87) Internationale Veröffentlichungsnummer: WO 1999/038528

(56) Entgegenhaltungen:
- WO-A-95/15768
- WO-A-97/30721
- BUSCHLE M ET AL: "Transloading of tumor antigen-derived peptides into antigen-presenting cells." PROCEEDINGS OF THE NATIONAL ACADEMY OF SCIENCES OF THE UNITED STATES OF AMERICA, (1997 APR 1) 94 (7) 3256-61. , XP002117665
- GIBSON T.D.: 'Protein Stabilisation using Additives Based on Multiple Electrostatic Interactions' BROWN F (ED): NEW APPROACHES TO STABILISATION OF VACCINES POTENCY. DEV BIOL STAND. Bd. 87, 1996, BASEL, Seiten 207 - 217

## Beschreibung

Die vorliegende Erfindung bezieht sich auf das Gebiet der Vakzinen.

Die immunogene Wirkung von traditionellen Vakzinen beruht zumeist auf abgetöteten oder abgeschwächten Krankheitserregern. In den traditionellen Vakzinen wirken die Verunreinigungen der Vakzine selbst oder andere Komponenten von Organismen als Adjuvantien, die die immunogene Wirkung des eigentlichen Antigens verstärken und/oder verlängern. Z.B. enthält der Diphtherie-Tetanus-Keuchhusten-Impfstoff zwei potente, von der Ganzzell-Keuchhusten-Vakzine stammende Adjuvantien (LPS = Lipopolysaccharid sowie PT = Pertussistoxin); ebenso haben die Ganzzell-Typhus- und Choleraimpfstoffe potente Adjuvantien (LPS sowie Choleratoxin); die BCG-Vakzine (Bacillus Calmette Guerin) hat starke nicht-spezifische immunstimulatorische Wirkungen.

Im Gegensatz zu den komplexen traditionellen Impfstoffen enthalten die modernen Vakzinen synthetische, rekombinante oder hochgereinigte Antigene in Form von Proteinen oder Peptiden. Diese Vakzinen gelten als sicherer, weisen jedoch im allgemeinen den Nachteil geringerer Immunogenität auf. Um diesen Nachteil zu kompensieren, werden den Vakzinen Adjuvantien beigegeben, um die spezifische Immunantwort auf Antigene zu verstärken und verlängern. Einige Adjuvantien haben die Eigenschaft, die T-Zellproliferation und die zelluläre Immunantwort zu verstärken.

Die meisten der bisher verwendeten Adjuvantien weisen jedoch Nebeneffekte auf, auch erfüllen diese Adjuvantien nicht die Anforderungen, die an die Sicherheit von Adjuvantien gestellt werden, wie Stabilität im Hinblick auf Adjuvanswirkung, minimale Toxizität ohne Wechselwirkung mit dem Antigen, ferner Abbaufähigkeit im Organismus sowie Fehlen einer eigenen immunogen Wirkung.

Eine Übersicht von gängigen Adjuvantien, die bisher für Vakzinen in Betracht gezogen wurden, wird von Vogel, 1995, und von Gupta und Siber, 1995, gegeben. Dazu zählen: anorganische Adjuvantien in Gelform (Aluminiumhydroxid/Aluminiumphosphat, Calciumphosphat); bakterielle Adjuvantien, wie Monophosphoryllipid A und Muramylpeptide, teilchenförmige Adjuvantien, wie die sog. ISCOMS ("immunostimulatory complexes"), Liposomen und bioabbaubare Mikrosphären, Adjuvantien auf der Grundlage von Ölemulsionen und Emulgatoren, wie Freund's Adjuvans oder IFA ("Incomplete Freund's Adjuvans"), Saponine (wie QS-21), Squalen; synthetische Adjuvantien, wie nicht-ionische Block-Copolymere, Muramylpeptidanaloge, synthetisches Lipid A, synthetische Polynukleotide und polykationische Adjuvantien, wie Polyarginin oder Polylysin (WO 97/30721).

Die Wahl eines Adjuvans stellt in der Regel einen Kompromiß dar, der das Ergebnis einer Abwägung zwischen Toxizität und Adjuvanswirkung der jeweiligen Substanz ist.

Bei Vakzineformulierungen wurde bisher im allgemeinen Bedacht auf Isotonizität genommen, die gängigen Vakzinformulierungen liegen üblicherweise in einer Salzkonzentration vor, die etwa 150 mM NaCl (ca. 300 mosmol/1) entspricht. Gängige Pufferformulierungen sind PBS und HBS (Phosphat-gepufferte bzw. HEPESgepufferte Salzlösung); z.B. wurde für eine ISCOM-Vakzine PBS pH 7.4 vorgeschlagen (Barr und Mitchell, 1996).

Der vorliegenden Erfindung lag die Aufgabe zugrunde, eine Vakzineformulierung bereitzustellen, die die Wirkung von Vakzinen auf der Grundlage von Antigenen in Form von Peptiden oder Proteinen verstärkt.

Es wurde überraschend festgestellt, daß die immunogene Wirkung einer adjuvanshältigen Vakzine auf Peptidbasis gesteigert wird, wenn die Vakzineformulierung eine niedrige Salzionenkonzentration aufweist bzw. frei von Salzen ist, und ein Zuckeralkohol enthält.

Die Erfindung betrifft somit eine Vakzine, enthaltend ein oder mehrere synthetische oder hochgereinigte natürliche Peptide oder Proteine als Antigen(e) sowie ein oder mehrere Adjuvantien dadurch gekennzeichnet, dass sie als Adjuvans ein Polykation enthält. Die Vakzine ist femoral dadurch gekennzeichnet, daß sie als Lösung oder Emulsion vorliegt, die frei von anorganischen Salzionen ist bzw. eine niedrige Salzionenkonzentration aufweist, und ein Zuckeralkohol enthält.

Im Zusammenhang mit der erfindungsgemäßen Vakzine wird unter "niedrige Salzionenkonzentration" eine Konzentration verstanden, die gleich oder niedriger ist als ca. 50% der Salzkonzentration einer isotonischen Lösung, was etwa ca. 75 mM Kochsalzlösung entspricht.

Bei der Berechnung der Ionenkonzentration ist zu berücksichtigen, daß im Fall der Verwendung von Peptid- bzw. Proteinantigenen, die als solche eine Ladung aufweisen, diese Ladung nicht in Rechnung gestellt wird.

Bevorzugt ist die Vakzine im wesentlichen frei von Natrium-, Chlorid- und Phosphationen, besonders bevorzugt ist sie im wesentlichen frei von sämtlichen anorganischen Salzionen ("im wesentlichen frei" bedeutet, daß der Vakzine keine Salze zugesetzt wurden, daß jedoch gegebenenfalls von Reagentien stammende Verunreinigungen oder Spuren von Ionen enthalten sein können; ebenfalls nicht eingerechnet werden von Adjuvantien stammende Ionen, z.B. bei Verwendung anorganischer Adjuvantien).

Für den Fall, daß die Vakzine, z.B. von Pufferlösung stammende, Phosphationen enthält, ist sie bevorzugt frei von Natrium- und Chloridionen. Für den Fall, daß sie Natrium- und/oder Chloridionen enthält, ist sie bevorzugt frei von Phosphationen.

In einer Ausführungsform der Erfindung enthält die Vakzine Antigen und Adjuvans in salzfreiem Medium, z.B. in destilliertem Wasser.

Die erfindungsgemäße Vakzine enthält als isotonisch machende Substanz ein Zuckeralkohol, wie Sorbit oder Mannit.

Die isotonisch machende Substanz liegt, je noch Molekulargewicht, bevorzugt in einer Konzentration vor, so daß die resultierende Lösung isotonisch oder leicht hypotonisch ist.

Bevorzugte Zuckeralkoholkonzentrationen liegen im Bereich von ca. 200 - 400 mM, insbesondere im Bereich von 250 - 300 mM. Die Osmolarität der Lösung beträgt zweckmäßig zwischen 200 - 400 mosmol/l, die Lösung kann aber auch stark hypotonisch sein.

Zusätzlich zur isotonisch machenden Substanz enthält die Lösung, in der die erfindungsgemäße Vakzine vorliegt, gegebenenfalls eine Puffersubstanz. Dafür kommen in erster Linie HEPES (N-[2-Hydroxyethyl] piperazine-N'-[2-ethanesulfonic acid]), oder TRIS (Tris[hydroxymethyl]aminomethane) in Betracht. Eine Puffersubstanz kann erforderlich sein, um die Vakzine auf einen physiologischen pH-Wert einzustellen, wenn die primäre Lösung vom physiologischen Wert abweicht.

Bezüglich der Peptid- bzw. Proteinantigene unterliegt die erfindungsgemäße Vakzine keinerlei Beschränkungen. Bei den Antigenen kann es sich um natürlich vorkommende immunogene Proteine, z.B von viralen oder bakteriellen Erregern stammende Proteine bzw. deren Fragmente oder zelluläre Abbauprodukte in Form von Peptiden handeln; oder um Tumorantigene bzw. Fragmente davon. In einer bevorzugten Ausführungsform ist das Antigen ein Tumorantigen bzw. ein davon abgeleitetes natürliches oder synthetisches Peptid, in diesem Fall liegt die Vakzine als Tumorvakzine vor.

Die Menge an wirksamem Antigen in der erfindungsgemäßen Vakzine kann über einen breiten Bereich variieren. Die Menge an Peptid hängt u.a. von der Verabreichungsart und der jeweiligen Formulierung ab. Die zu verabreichende Menge an Peptid kann ca. 0.1 µg bis ca. 10000 µg pro Vakzinierungsdosis betragen, im allgemeinen 1.0 µg bis ca. 1000 µg, insbesondere ca. 10 µg bis ca. 500 µg.

Gemäß der Erfindung ist das Adjuvans eine Substanz, wie sie in der WO 97/30721, auf deren Offenbarung hiermit ausdrücklich Bezug genommen wird, als Zusatz für Protein- bzw. Peptidvakzine vorgeschlagen wurde, ein Polykation, wie Polyarginin oder Polylysin, das gegebenenfalls modifiziert ist, z.B. mit einem Zuckerrest.

Im Rahmen der vorliegenden Erfindung wurde überraschend festgestellt, daß eine salzfreie, mit Sorbit isotonisch gemachte Tumorvakzine, enthaltend ein MHC-bindendes, von einem Tumorantigen abgeleitetes Peptid sowie Polyarginin als Adjuvans, gegenüber einer hinsichtlich Peptid/Adjuvans identischen, herkömmlich formulierten, d.h. eine isotonische Salzkonzentration enthaltenden Tumorvakzine eine stärkere Antitumoraktivität aufweist. Es wurde festgestellt, daß die Peptide zusammen mit dem Adjuvans in Sorbitlösung besser löslich sind als in herkömmlichem PBS Puffer. Ohne auf die Theorie festgelegt sein zu wollen, dürfte die verbesserte Wirkung der Vakzine, neben der verbesserten Löslichkeit, darauf zurückzuführen sein, daß die Interaktion zwischen Peptid und Adjuvans erleichtert und damit die Wirkung des Adjuvans verstärkt wird. Gegebenfalls ist die verbesserte Wirkung der Vakzine außerdem auf eine Co-Adjuvans-Wirkung der isotonisch machenden Substanz, z.B. Sorbit, zurückzuführen, d. h. diese Substanz (Sorbit) hat als solche eine gewisse Adjuvanswirkung, die die Wirkung des primären Adjuvans verstärkt.

Um eine Vakzine optimal zu formulieren, wird zweckmäßig wie folgt vorgegangen: ausgehend von einem definierten Antigen, das die gewünschte Immunantwort hervorrufen soll, wird in einem ersten Schritt ein auf das Antigen abgestimmtes Adjuvans ermittelt, wie in der Fachliteratur, insbesondere in der WO 97/30721, beschrieben. In einem nächsten Schritt wird die Vakzine dahingehend optimiert, daß der Antigen/Adjuvansmischung bei ansonsten identischer Zusammensetzung unterschiedliche isotonisch machende Substanzen im Sinne der Definition der vorliegenden Erfindungen, d.h. Zuckeralkohole, in isotonischer bzw. leicht hypotonischer Konzentration zugesetzt werden und die Lösung auf einen physiologischen pH-Wert im Bereich von pH 4.0 bis 10.0, insbesondere 7.4, gebracht wird.

Eine weitere Möglichkeit zur Testung der Effizienz einer Formulierung ist die Verwendung eines *in vitro* Modellsystems. Hierbei werden APCs zusammen mit Adjuvans, Peptid und Kandidatensubstanz inkubiert und die relative Aktivierung eines T-Zellklons, der das verwendete Peptid spezifisch erkennt, gemessen (Coligan et al.,1991; Lopez et al.,1993)

Die Effizienz der Formulierung kann gegebenenfalls auch über die zelluläre Immunantwort durch den Nachweis einer "delayed-type hypersensitivity" (DTH)-Reaktion in immunisierten Tieren gezeigt werden.

Letztlich wird die immunmodulatorische Wirkung der Formulierung im Tierversuch gemessen. Im Falle einer Tumorvakzine, wie im vorliegenden Beispiel, können u.a. etablierte Tumormodelle, bei denen von Immunzellen erkannte Peptidsequenzen bekannt sind, eingesetzt werden. Die Vakzine, enthaltend bei konstanter Peptid/Adjuvans-Zusammensetzung unterschiedliche Puffersubstanzen, wird den Versuchstieren appliziert. Der Schutz vor Tumorwachstum ist ein Maß für die Wirksamkeit einer Tumorvakzine.

### Beispiel

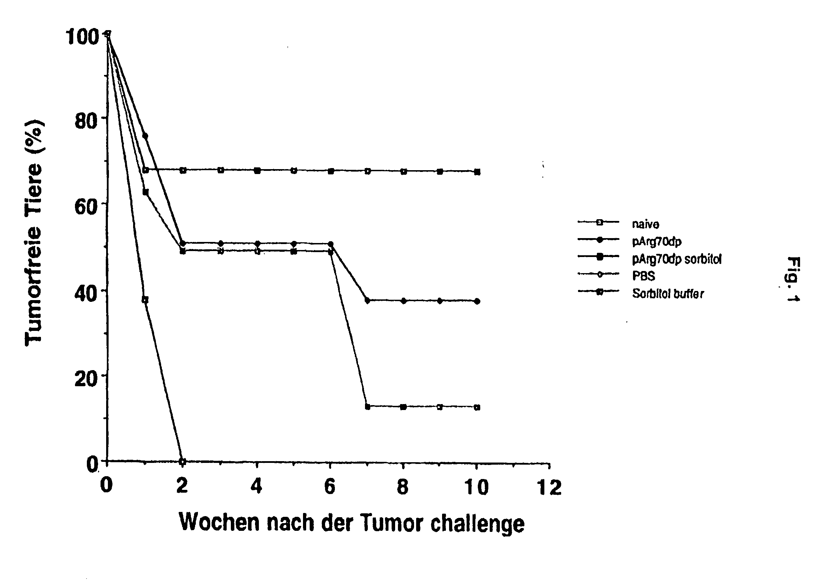

Die Versuche wurden durchgeführt, wie in der WO 97/30721 beschrieben.
a) DBA/2 Mäuse wurden mit einem Gemisch aus 100 µg MHC Klasse I bindendem Peptid SYFPETHI (Bezeichnung "P815 JAK1") und 75 µg Polyarginin (Polymerisationsgrad 70, SIGMA Chemicals, St. Louis MO) pro Tier dreimal in je einwöchigem Abstand geimpft. Die Peptid/Adjuvantslösung wurde in Sorbitlösung (270 mM Sorbit, 5 mM HEPES) oder phosphatgepufferter Kochsalzlösung (PBS, GIBCO BRL) verabreicht. Kontrollmäuse erhielten entweder 100 µg Peptid/Tier ohne Adjuvants in Sorbitpuffer oder wurden nicht vakziniert. Eine Woche nach der letzten Vakzinierung wurden 10⁴ viable Tumorzellen injiziert und Tumorwachstum wöchentlich festgehalten.
   Das Ergebnis der Versuche ist in Fig. 1 dargestellt. Die Abbildung zeigt den Vergleich der Effizienz der P815 JAK1-Vakzine in Sorbitlösung gegen eine Vakzine in gepufferter, isotonischer Salzlösung im Tiermodell. Es zeigte sich, daß Tiere, die die Vakzine in Sorbitlösung erhielten, besser geschützt sind als Mäuse, die mit Peptid/polyArginin in PBS geimpft wurden.
b) Für die Löslichkeitsversuche wurden Gemische aus fluoreszenzmarkiertem Peptid LFEAIEGFI oder GYKDGNEYI hergestellt: 100 µg fluoreszenzmarkiertes Peptid wurde mit 75 µg Polyarginin (Arg; Polymerisationsgrad 70, SIGMA Chemicals, St. Louis MO) entweder in Sorbitlösung oder HEPES-gepufferter Kochsalzlödung (HBS: 20 mM HEPES pH 7.5, 150 mM NaCl) versetzt. Nach drei Stunden wurde die Menge an gelöster Fluoreszenz durch Bestimmung der Extinktion bei 490 nM gemessen. Als Testprotein wurde das Green Fluorescent Protein verwendet.

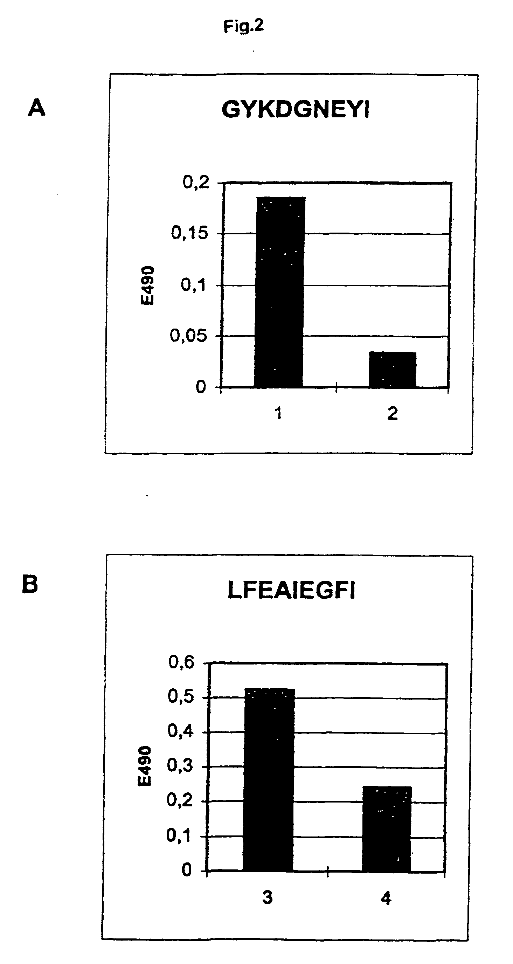

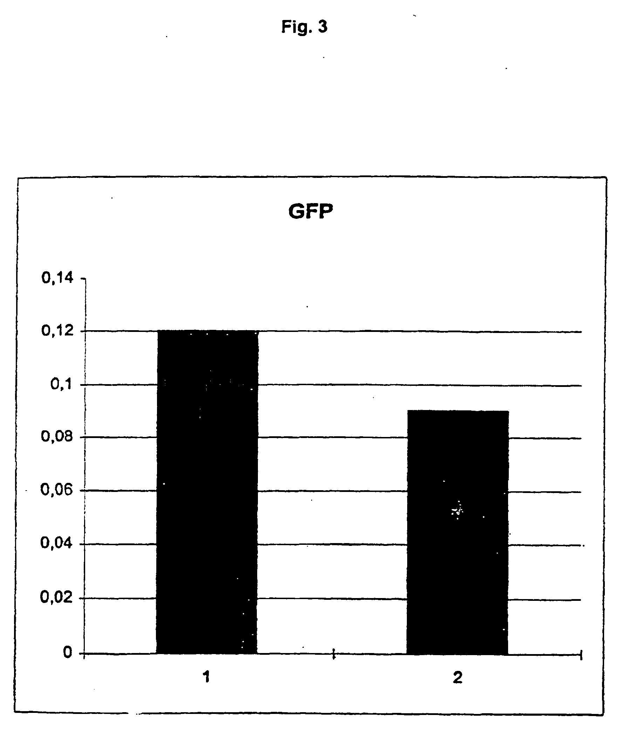

Fig. 2 und Fig. 3 zeigen den Vergleich der Löslichkeit der Komplexe nach Mischung in gepufferter Salzlösung oder Sorbitlösung. Die beiden fluoreszenzmarkierten Peptide (Fig. 2A und Fig. 2B) und das Green Fluorescent Protein (GFP; ca. 30 Kd; Fig. 3) wurden in diesen Versuch einbezogen. Durch Anmischung der Vakzine in Sorbitlösung ergab sich eine deutlich verbesserte Löslichkeit und Recovery (erhöhte Fluoreszenz) sowohl mit den beiden getesteten Peptiden als auch mit GFP.

### Literatur

Abraham, E., 1992, Vaccine 10, 461-468

Allison, A.C., und Byars, N.E., 1991, Mol Immunol 28, 279-284

Azuma, I., 1992, Vaccine 10, 1000-1004

Baker, P.J., et al., 1988, Infect Immun 56, 3064-3066

Coligan, J.E. et al., 1991, Current Protocols in Immunology, Wiley, New York

Ellouz, F., et al., 1974, Biochem Biophys Res Commun 59, 1317-1325

Gupta, R.K. und Siber G.R., 1995, Vaccine 13, 1263-1276

Gregoriadis, G., 1990, Immunol Today 11, 89-97

Harrington, D.G., et al., 1978, Infect Immun 24, 160-166

Hunter, R., et al., 1991, Vaccine 9, 250-255

Lopez, J.A., et al., 1993, Eur. J. Immunol. 23, 217-223

Marx, P.A., et al., 1993, Science 28, 1323-1327

Mbawuike, I.N., et al., 1990, Vaccine 8, 347-352

Mowat, A.M., und Donachie, A.M., 1991, Immunol Today 12, 383-385

Newman, M.J., et al., 1992, J Immunol 148, 23572362

Phillips, N.C. und Emili, A, 1992, Vaccine 10, 151-158

Rarrtmensee, H.G., et al., 1995, Immunogenetics 41, 178-228

Relyvelt, E.H., 1986, Develop Biol Standard, 65, 131-136

Ribi, E., 1984, J Biol Res Mod, 3, 1-9

Stuart-Harris, C.H., 1969, Bull WHO 41, 617-621

Takahashi, H., et al., 1990, Nature 344, 873-875

Thapar, M.A., et al., 1991, Vaccine 9, 129-133

Vogel, F. R. 1995, Ann N Y Acad Sci 754, 153-160

Warren, H.S., et al., 1986, Ann Rev Immunol 4, 369-388

Waters, R.V., et al., 1986, Infect Immun 52, 816-825

## Patentansprüche

1. Vakzine, enthaltend ein oder mehrere synthetische oder hochgereinigte natürliche Peptide oder Proteine als Antigen(e) sowie ein oder mehrere Adjuvantien, **dadurch gekennzeichnet, dass** sie als Adjuvans ein Polykation enthält und als Lösung bzw. Emulsion vorliegt, die
a) frei von anorganischen Salzionen ist bzw. eine niedrige Konzentration anorganischer Ionen, entsprechend einer Konzentration, die gleich oder niedriger ist als die einer ca. 75 mM Kochsalzlösung, aufweist, und die
b) einen Zuckeralkohol enthält.

2. Vakzine nach Anspruch 1, **dadurch gekennzeichnet, dass** sie im wesentlichen frei von sämtlichen anorganischen Salzionen ist.

3. Vakzine nach Anspruch 1, **dadurch gekennzeichnet, dass** der Zuckeralkohol Sorbit ist.

4. Vakzine nach Anspruch 1, **dadurch gekennzeichnet, dass** der Zuckeralkohol Mannit ist.

5. Vakzine nach Anspruch 1, **dadurch gekennzeichnet, dass** der Zuckeralkohol in einer Konzentration vorliegt, so dass die resultierende Lösung isotonisch oder leicht hypotonisch ist.

6. Vakzine nach Anspruch 5, **dadurch gekennzeichnet, dass** die Zuckeralkoholkonzentrationen im Bereich von ca. 200 - 400 mM liegt.

7. Vakzine nach Anspruch 6, **dadurch gekennzeichnet, dass** die Konzentration 250 - 300 mM beträgt.

8. Vakzine nach einem der Ansprüche 1 bis 7, **dadurch gekennzeichnet, dass** sie zusätzlich eine Puffersubstanz enthält.

9. Vakzine nach einem der Ansprüche 1 bis 8, **dadurch gekennzeichnet, dass** sie als Antigen ein Peptid enthält.

10. Vakzine nach Anspruch 9, **dadurch gekennzeichnet, dass** das Peptid von einem Tumorantigen abgeleitet ist.

11. Vakzine nach einem der Ansprüche 1 bis 10, **dadurch gekennzeichnet, dass** sie als Adjuvans Polyarginin enthält.

## Claims

1. Vaccine containing one or more synthetic or highly purified natural peptides or proteins as antigen(s) as well as one or more adjuvants, **characterised in that** it contains a polycation as adjuvant and is present as a solution or emulsion which is
a) free from inorganic salt ions or has a low concentration of inorganic ions, corresponding to a concentration which is equal to or less than that of an approximately 75 mM saline solution, and
b) contains a sugar alcohol.

2. Vaccine as claimed in Claim 1, **characterised in that** it is substantially free of all inorganic salt ions.

3. Vaccine as claimed in Claim 1, **characterised in that** the sugar alcohol is sorbitol.

4. Vaccine as claimed in Claim 1, **characterised in that** the sugar alcohol is mannitol.

5. Vaccine as claimed in Claim 1, **characterised in that** the sugar alcohol is present in a concentration such that the resulting solution is isotonic or slightly hypotonic.

6. Vaccine as claimed in Claim 5, **characterised in that** the sugar alcohol concentration is in the range from approximately 200-400 mM.

7. Vaccine as claimed in Claim 6, **characterised in that** the concentration is 250-300 mM.

8. Vaccine as claimed in any one of Claims 1 to 7, **characterised in that** it additionally contains a buffer substance.

9. Vaccine as claimed in any one of Claims 1 to 8, **characterised in that** it contains a peptide as antigen.

10. Vaccine as claimed in Claim 9, **characterised in that** the peptide is derived from a tumour antigen.

11. Vaccine as claimed in any one of Claims 1 to 10, **characterised in that** it contains polyarginine as adjuvant.

## Revendications

1. Vaccin contenant un ou plusieurs peptides ou protéines synthétiques ou naturel(le)s hautement purifié(e)s comme antigène(s) ainsi qu'un ou plusieurs adjuvants, **caractérisé en ce qu'**il contient un polycation à titre d'adjuvant et se présente sous forme de solution ou d'émulsion, qui
a) est exempte de sels d'ions inorganiques ou présente une faible concentration en ions inorganiques, conformément à une concentration qui est identique ou plus faible que celle d'une solution de sel de cuisine à environ 75 mM, et qui
b) contient un alcool de sucre.

2. Vaccin selon la revendication 1, **caractérisé en ce qu'**il est sensiblement exempt de tous sels d'ions inorganiques.

3. Vaccin selon la revendication 1, **caractérisé en ce que** l'alcool de sucre est le sorbitol.

4. Vaccin selon la revendication 1, **caractérisé en ce que** l'alcool de sucre est le mannitol.

5. Vaccin selon la revendication 1, **caractérisé en ce que** l'alcool de sucre est présent en une concentration qui permet à la solution résultante d'être isotonique ou légèrement hypotonique.

6. Vaccin selon la revendication 5, **caractérisé en ce que** les concentrations en alcool de sucre se situent dans la plage allant d'environ 200 à 400 mM.

7. Vaccin selon la revendication 6, **caractérisé en ce que** la concentration atteint 250 à 300 mM.

8. Vaccin selon l'une des revendications 1 à 7, **caractérisé en ce qu'**il comprend en plus une substance tampon.

9. Vaccin selon l'une quelconque des revendications 1 à 8, **caractérisé en ce qu'**il contient un peptide comme antigène.

10. Vaccin selon la revendication 9, **caractérisé en ce que** le peptide est dérivé d'un antigène tumoral.

11. Vaccin selon l'une quelconque des revendications 1 à 10, **caractérisé en ce qu'**il contient de la polyarginine à titre d'adjuvant.
